# EUROPÄISCHE PATENTSCHRIFT

(11) **EP 1 853 724 B1**
(45) Veröffentlichungstag und Bekanntmachung des Hinweises auf die Patenterteilung: **21.10.2009**
(21) Anmeldenummer: 06707458.3
(22) Anmeldetag: 02.03.2006
(51) Int. Cl.: C12Q 1/68

(54) **VERFAHREN ZUR UNTERSUCHUNG VON CYTOSIN-METHYLIERUNGEN IN DNA**
METHOD FOR INVESTIGATING CYTOSINE METHYLATIONS IN DNA
PROCÉDÉ D'ANALYSE DES MÉTHYLATIONS DE LA CYTOSINE DANS L'ADN

(30) Priorität: 03.03.2005 DE 102005011398
(43) Veröffentlichungstag der Anmeldung: 14.11.2007
(73) Patentinhaber: Epigenomics AG, 10178 Berlin (DE)
(72) Erfinder: LEWIN, Jörn, 10115 Berlin (DE); DISTLER, Jürgen, 12163 Berlin (DE); LESCHE, Ralf, 13156 Berlin (DE); SCHUSTER, Matthias, 13156 Berlin (DE)
(86) Internationale Anmeldenummer: PCT/EP2006/002092
(87) Internationale Veröffentlichungsnummer: WO 2006/092339

(56) Entgegenhaltungen:
- WO-A-02/38811
- WO-A-03/000926
- WO-A-03/027259
- WO-A-03/064700
- FOJTOVA MILOSLAVA ET AL: "Cytosine methylation of plastid genome in higher plants. Fact or artefact?" PLANT SCIENCE (SHANNON), Bd. 160, Nr. 4, März 2001 (2001-03), Seiten 585-593, XP002382695 ISSN: 0168-9452
- RAND KEITH ET AL: "Conversion-specific detection of DNA methylation using real-time polymerase chain reaction (ConLight-MSP) to avoid false positives." METHODS (SAN DIEGO, CALIF.) JUN 2002, Bd. 27, Nr. 2, Juni 2002 (2002-06), Seiten 114-120, XP002382696 ISSN: 1046-2023
- ROTHMAN RICHARD E ET AL: "Detection of bacteremia in emergency department patients at risk for infective endocarditis using universal 16S rRNA primers in a decontaminated polymerase chain reaction assay." THE JOURNAL OF INFECTIOUS DISEASES. 1 DEC 2002, Bd. 186, Nr. 11, 1. Dezember 2002 (2002-12-01), Seiten 1677-1681, XP002382697 ISSN: 0022-1899
- SASAKI M ET AL: "Bisulfite conversion-specific and methylation-specific PCR: a sensitive technique for accurate evaluation of CpG methylation" BIOCHEMICAL AND BIOPHYSICAL RESEARCH COMMUNICATIONS, ACADEMIC PRESS INC. ORLANDO, FL, US, Bd. 309, Nr. 2, 19. September 2003 (2003-09-19), Seiten 305-309, XP004451513 ISSN: 0006-291X
- XIONG Z AND LAIRD P W: "COBRA: a sensitive and quantitative DNA methylation assay" NUCLEIC ACIDS RESEARCH, OXFORD UNIVERSITY PRESS, SURREY, GB, Bd. 25, Nr. 12, 1997, Seiten 2532-2534, XP002106407 ISSN: 0305-1048

## Beschreibung

### Hintergrund der Erfindung

Die vorliegende Erfindung betrifft ein Verfahren zum Nachweis von 5-Methylcytosin in DNA. 5-Methylcytosin ist die häufigste kovalent modifizierte Base in der DNA eukaryontischer Zellen. Sie spielt eine wichtige biologische Rolle, u.a. bei der Transkriptionsregulation, beim genetischen Imprinting und in der Tumorgenese (zur Übersicht: Millar et al.: Five not four: History and significance of the fifth base. In: The Epigenome, S. Beck and A. Olek (eds.), Wiley-VCH Verlag Weinheim 2003, S. 3-20). Die Identifizierung von 5-Methylcytosin als Bestandteil genetischer Information ist daher von erheblichen Interesse. Ein Nachweis der Methylierung ist allerdings schwierig, da Cytosin und 5-Methylcytosin das gleiche Basenpaarungsverhalten aufweisen. Viele der herkömmlichen, auf Hybridisierung beruhenden Nachweisverfahren vermögen daher nicht zwischen Cytosin und Methylcytosin zu unterscheiden. Zudem geht die Methylierungsinformation bei einer PCR-Amplifikation vollständig verloren.

Die herkömmlichen Methoden zur Methylierungsanalyse arbeiten im wesentlichen nach zwei unterschiedlichen Prinzipien. Zum einen werden methylierungsspezifische Restriktionsenzyme benutzt, zum anderen erfolgt eine selektive chemische Umwandlung von nicht-methylierten Cytosinen in Uracil (sog.: Bisulfit-Behandlung, siehe etwa: DE 101 54 317 A1; DE 100 29 915 A1).

Da die Behandlung mit methylierungsspezifischen Restriktionsenzymen durch die Sequenzspezifität der Enzyme auf bestimmte Sequenzen beschränkt ist, wird für die meisten Anwendungen eine Bisulfit-Behandlung durchgeführt (zur Übersicht DE 100 29 915 A1 S.2, Zeilen 35-46). Die chemisch vorbehandelte DNA wird dann meist amplifiziert und kann auf unterschiedliche Weise analysiert werden (zur Übersicht: WO 02/072880 S. 1 ff). Von großem Interesse sind dabei Verfahren, die in der Lage sind, Methylierung sensitiv und quantitativ zu detektieren. Dies gilt aufgrund der wichtigen Rolle der Cytosin-Methylierung in der Krebsentstehung insbesondere in Hinblick auf diagnostische Anwendungen. Von besonderer Bedeutung sind dabei Methoden, die es erlauben, abweichende Methylierungsmuster in Körperflüssigkeiten, etwa in Serum, nachzuweisen. Denn anders als die instabile RNA ist DNA oft in Körperflüssigkeiten anzutreffen. Bei destruktiven pathologischen Prozessen wie Krebserkrankungen ist die DNA-Konzentration im Blut sogar erhöht. Eine Krebsdiagnostik über eine Methylierungsanalyse von in Körperflüssigkeiten befindlicher Tumor-DNA ist also möglich und schon mehrfach beschrieben (siehe etwa: Palmisano et al.: Predicting lung cancer by detecting aberrant promoter methylation in sputum. Cancer Res. 2000 Nov 1;60(21):5954-8). Ein besonderes Problem besteht jedoch darin, dass sich in den Körperflüssigkeiten neben der DNA mit dem krankheitstypischen Methylierungsmuster eine große Menge an DNA der gleichen Sequenz aber eines anderen Methylierungsmusters befindet. Die Diagnoseverfahren müssen daher in der Lage sein, geringe Mengen besonders methylierter DNA vor einem starken Hintergrund an DNA derselben Sequenz aber eines anderen Methylierungsmusters (im folgenden: Hintergrund-DNA) nachzuweisen.

WO 03/000926 stellt ein solches Verfahren zur Elimination von Hintergrund-DNA dar. Hier wird die nicht-methylierte DNA untersucht während die methylierte DNA abgetrennt werden soll. Wie aus Figur 2 ersichtlich, wird auf der linken Seite die nicht zu analysierende Hintergrund-DNA dargestellt, die in diesem Beispiel partiell methyliert vorliegt (schwarz ausgefüllte Dreiecke). Auf der rechten Seite befindet sich entsprechend unmethylierte, zu analysierende DNA. Im ersten Schritt (A) findet eine Bisulfit-Umwandlung statt, im zweiten Schritt (B) erfolgt die erste Amplifikation. Im dritten Schritt (C) wird die nicht zu analysierende Hintergrund-DNA gespalten. Bei der gespaltenen Hintergrund DNA handelt es sich also um die methylierte DNA. Die verbleibende DNA wird anschliessend erneut amplifiziert. Ihre Sequenzeigenschaften erlauben nachfolgend Rückschlüsse auf den Methylierungsstatus der zu untersuchenden DNA an prinzipiell allen Positionen, die sich in dem amplifizierten Bereich befinden. Zusammengefasst wird in WO 03/000926 die DNA (1.) zunächst chemisch mit Bisulfit umgewandelt und danach (2.) mit Restriktionsenzymen geschnitten, welche spezifisch die methylierten CG Positionen erkennen. Letztere ist dann in WO 03/000926 die Hintergrund-DNA, während die nicht-methylierte, C/U bzw. T-umgewandelte DNA weiter analysiert wird. Die Reihenfolge ist somit umgekehrt zur vorliegenden Anmeldung.

Die herkömmlichen Verfahren zur Methylierungsanalyse lösen dieses Problem nur eingeschränkt. Üblicherweise wird die chemisch vorbehandelte DNA mittels eines PCR-Verfahrens amplifiziert. Über die Verwendung methylierungsspezifischer Primer oder Blocker soll dann eine selektive Amplifikation nur der methylierten (bzw. bei umgekehrten Ansatz: unmethylierten) DNA gewährleistet werden. Der Einsatz methylierungsspezifischer Primer ist als sog. "methylierungssensitive PCR" bekannt ("MSP"; Herman et al.: Methylation-specific PCR: a novel PCR assay for methylation status of CpG islands. Proc Natl Acad Sci U S A. 1996 Sep 3;93(18):9821-6). Ein vergleichbar sensitives Verfahren ist die sogenannte "Heavy Methyl"-Methode. Dabei wird eine spezifische Amplifizierung nur der ursprünglich methylierten (bzw. unmethylierten) DNA durch Einsatz von methylierungsspezifischen Blocker-Oligomeren erreicht (zur Übersicht: WO 02/072880). Sowohl MSP wie Heavy Methyl sind als quantifizierbare Real-Time-Varianten anwendbar. Diese ermöglichen es, den Methylierungsstatus weniger Positionen direkt im Verlauf der PCR nachzuweisen, ohne dass eine nachfolgende Analyse der Produkte erforderlich wäre ("MethyLight" - WO00/70090; US 6,331,393). Eine Ausführungsform ist dabei das "Taqman"-Verfahren. Dieses verwendet Sondenmoleküle, die ein Fluoreszenzfarbstoff-Quencher-Paar tragen. Die Sonden hybridisieren sequenzspezifisch an die Amplifikate und werden im Zuge des nächsten Amplifikationszyklus durch die Exonuklease-Aktivität der Polymerase abgebaut. Durch die Trennung von Quencher und Farbstoff entsteht ein detektierbares Fluoreszenz-Signal (siehe etwa Eads et al.: MethyLight: a high-throughput assay to measure DNA methylation. Nucleic Acids Res. 2000 Apr 15;28(8):E32). Eine weitere MethyLight-Ausführungsform ist das sog. Lightcycler-Verfahren. Dabei werden zwei unterschiedliche Sonden eingesetzt, die in unmittelbarer Nähe zueinander an das Amplifikat hybridisieren, und die dann über Fluoreszenz-Resonanz-Energie-Transfer (FRET) ein detektierbares Signal erzeugen.

Die Anwendbarkeit dieser Verfahren für einen sensitiven und spezifischen Nachweis methylierter DNA vor einem großen Hintergrund an unmethylierter DNA ist allerdings beschränkt. So besteht die Gefahr, dass es über eine unspezifische Amplifikation von Hintergrund-DNA zu falsch-positiven Ergebnissen kommt. Falsch positive Signale stellen jedoch eines der signifikantesten Probleme des Einsatzes der Methylierungstechnologie für die Krebs-Früherkennung dar. Eine Erhöhung der Spezifität des Methylierungsnachweises bedeutet daher einen bedeutsamen Schritt für die Entwicklung entsprechender Früherkennungstests. Eine zuverlässige, kommerzielle Nutzung der Methylierungsanalyse im Bereich der Tumor-Frühdiagnostik wird so erleichtert.

Um die Spezifität des Methylierungsnachweises zu erhöhen, verwenden die bekannten Verfahren für die Amplifiktion Primer- oder Blockersequenzen, in denen mehrere methylierungsspezifische Positionen enthalten sind. Diese Sequenzanforderungen erlauben allerdings nur einen Nachweis von Sequenzen, in denen in einem engen Sequenzbereich eine Vielzahl von CpG-Positionen auftreten. Diese Sequenzanforderungen schränken die Anwendbarkeit der Verfahren ein.

Aufgrund der genannten besonderen biologischen und medizinischen Bedeutung der Cytosin-Methylierung und aufgrund der oben erwähnten Nachteile des Standes der Technik besteht ein großes technisches Bedürfnis an der Entwicklung leistungsfähiger Methoden zur sensitiven und spezifischen Methylierungsanalyse. Im folgenden ist ein überraschend einfaches Verfahren beschrieben, mit dem die Spezifität des Methylierungsnachweises erhöht werden kann.

Erfindungsgemäß wird vor der Bisulfitumwandlung und der anschließenden Amplifikation ein enzymatischer Filterschritt durchgeführt. Durch diesen Filter wird die Hintergrund DNA aus dem Reaktionsgemisch entfernt. In diesem Filterschritt wird ein Gemisch aus unterschiedlichen methylierungsspezifischen Retriktionsenzymen eingesetzt. Nach dem Restriktionsverdau erfolgen in herkömmlicher Weise Bisulfitumwandlung und Amplifikation/Detektion. Der enzymatische Abbau der Hintergrund-DNA verringert die Gefahr falsch positiver Ergebnisse und erlaubt so einen spezifischeren Nachweis von methylierten Cytosinpositionen.

Zwar ist sowohl der Einsatz von methylierungsspezifschen Restriktionsenzymen wie auch der Einsatz der Bisulfitumwandlung in der Methylierungsanalyse seit langem bekannt (s.o.). Eine Kombination aus methylierungsspezifischem Restriktionsverdau mit einer Bisulfitumwandlung und einer anschließenden Amplifikation ist bisher jedoch noch nirgends beschrieben. Aufgrund der besonderen biologischen und medizinischen Bedeutung der Cytosin-methylierung und aufgrund der Nachteile der bekannten Verfahren stellt das Eröffnen dieser vorteilhaften, neuen und überraschend einfachen Technologie einen wichtigen technischen Fortschritt dar.

### Beschreibung

Das erfindungsgemäße Verfahren zur Methylierungsanalyse verläuft in folgenden Schritten:
1) aus einer biologischen Probe wird DNA isoliert,
2) die DNA wird mit Hilfe mindestens eines methylierungsspezifischen Restriktionsenzyms umgesetzt, wobei das methylierungsspezifische Restriktionsenzym die Hintergrund DNA abbaut,
3) die DNA wird chemisch oder enzymatisch umgewandelt, wobei unmethyliertes Cytosin in Thymin oder eine andere Base überführt wird, die sich im Basenpaarungsverhalten von Cytosin unterscheidet, während Methylcytosin unverändert bleibt,
4) die umgewandelte DNA wird analysiert.

Im ersten Schritt des erfindungsgemäßen Verfahrens wird die DNA aus einer biologischen Probe isoliert. Dabei kann die zu untersuchende DNA je nach diagnostischer oder wissenschaftlicher Fragestellung aus unterschiedlichen Quellen stammen. Für diagnostische Fragestellungen dienen als Ausgangsmaterial bevorzugt Gewebeproben, aber auch Körperflüssigkeiten, insbesondere Serum. Möglich ist auch, die DNA aus Sputum, Stuhl, Urin oder Gehirn-Rückenmarks-Flüssigkeit zu verwenden. Die DNA-Isolierung kann nach Standardverfahren erfolgen, aus Blut etwa unter Verwendung des Qiagen UltraSens DNA Extraktions-Kits.

Im zweiten Schritt des erfindungsgemäßen Verfahrens wird die DNA mit Hilfe mindestens eines methylierungsspezifischen Restriktionsenzyms umgesetzt. Dabei baut das methylierungsspezifische Restriktionsenzym die Hintergrund DNA ab. Dem Fachmann sind eine Vielzahl von Restriktionsenzymen bekannt, die erfindungsgemäß einsetzbar sind. Insbesondere bietet die REBASE-Datenbank (http://rebase.neb.com/) vielfältige Informationen zu methylierungssensitiven Restriktionsenzymen. Bevorzugt ist die Verwendung der folgender Enyzme: HpyCH4 IV, Hha I, Hpa II; HinP1I; Aci I, Zra I, SNAB1, Sal I; Pml1, PaeR7I, Cla I, BspDI, BsaAI, Ava I Die Schnittstellen dieser Enzyme sind in Tabelle 1 aufgeführt. Alle Enzyme sind kommerziell erhältlich, etwa über New England Biolabs (www.neb.com). Die Reaktionsbedingungen der enzymatischen Umsetzung sind Stand der Technik und ergeben sich etwa aus den von den Herstellern gelieferten Protokollen.

Die Enzyme haben unterschiedliche lange Erkennungssequenzen. Dem Fachmann ist bekannt, dass er durch Auswahl der Enzyme die Häufigkeit der Fragmentierung beeinflussen kann. Wählt er Enzyme, die eine Vierersequenz erkennen, so kommt es zu einer deutlich häufigeren Schnitten als bei Verwendung von Enzymen, die eine längere Erkennungssequenz besitzen.

In einer bevorzugten Ausführungsform erfolgt die Umsetzung mittels einer Mischung von unterschiedlichen Restriktionsenzymen. So ist gewährleistet, dass die Hintergrund DNA möglichst vollständig fragmentiert wird, und so in der anschließenden Amplifikation als Template nicht mehr zur Verfügung steht.

In einer bevorzugten Ausführungsform ist die Enzymmischung so zusammengestellt, dass alle verwendeten Enzyme bei den gewählten Puffer- und Reaktionsbedingungen aktiv sind.

Im dritten Schritt des erfindungsgemäßen Verfahrens wird die enzymatisch umgesetzte DNA chemisch oder enzymatisch umgewandelt, wobei unmethyliertes Cytosin in Thymin oder eine andere Base überführt wird, die sich im Basenpaarungsverhalten von Cytosin unterscheidet, während Methylcytosin unverändert bleibt. Bevorzugt wird dabei eine chemische "Bisulfitbehandlung" durchgeführt. Die Bisulfitumwandlung ist dem Fachmann in unterschiedlichen Variationen bekannt (siehe etwa: Frommer et al.: A genomic sequencing protocol that yields a positive display of 5-methylcytosine residues in individual DNA strands. Proc Natl Acad Sci U S A. 1992 Mar 1;89(5):1827-31; Olek, A modified and improved method for bisulphite based cytosine methylation analysis. Nucleic Acids Res. 1996 Dec 15;24(24):5064-6.; DE 100 29 915; DE 100 29 915). Besonders bevorzugt erfolgt die Bisulfitumwandlung in Gegenwart von denaturierenden Lösemittein, etwa Dioxan, und eines Radikalfängers (vgl.: DE 100 29 915). Weitere bevorzugte Ausführungsformen der Bisulfitumwandlung sind in den deutschen Patentanmeldungen DE 103 47 396.3; DE 103 47 397.1; DE 103 47 400.5 und DE 103 47 399.8 beschrieben.

In einer anderen bevorzugten Ausführungsform wird die DNA nicht chemisch, sondern enzymatisch umgewandelt. Dies ist etwa durch Einsatz von Cytidin-Deaminasen denkbar, die unmethylierte Cyidine schneller umsetzen als methylierte Cytidine. Ein entsprechendes Enzym ist kürzlich identifiziert worden (Bransteitter et al.: Activation-induced cytidine deaminase deaminates deoxycytidine on single-stranded DNA but requires the action of RNase. Proc Natl Acad Sci U S A. 2003 Apr 1;100(7):4102-7; vgl.: Deutsche Patentanmeldung: 103 31 107.6).

Im letzten Schritt des erfindungsgemäßen Verfahrens wird die umgewandelte DNA analysiert und daraufhin auf den Methylierungsstatus der ursprünglichen DNA geschlossen. Die umgewandelte DNA kann anhand der gängigen molekularbiologischen Verfahren analysiert werden, etwa über Hybidisierung oder Sequenzierung. In bevorzugten Variante, in denen der Methylierungsstatus möglichst sensitiv nachgewiesen werden soll, wird die umgewandelte DNA zunächst amplifiziert. Hierzu sind dem Fachmann unterschiedliche Verfahren bekannt, etwa Ligasekettenreaktionen. In einer bevorzugten Ausführungsform wird die DNA allerdings über eine Polymerasereaktion amplifiziert. Hierzu sind verschiedene Ausgestaltungen denkbar, etwa die Verwendung isothermer Amplifikationsverfahren. Besonders bevorzugt sind allerdings Polymerasekettenreaktionen (PCR). In einer ganz besonders bevorzugten Ausführungsform erfolgt die PCR unter Verwendung von Primern, die spezifisch nur an Positionen der umgewandelten Sequenz binden, die vorher entweder methyliert (oder bei umgekehrtem Ansatz: unmethyliert) waren. Dieses Verfahren ist bei bisulfitierter DNA unter dem Namen methylierungssensitive PCR bekannt (MSP). Dabei werden Primer verwendet, die mindestens ein 5'- CpG -3' Dinukleotid enthalten; bevorzugt sind Primer, die mindestens drei 5'- CpG- 3'-Positionen tragen, von denen mindestens eine am 3' Ende lokalisiert ist. Für die Amplifikation der unmethylierten Sequenzen bzw. der Gegenstränge sind dementsprechend 5'-TG-3' oder 5'-CA-3'-Dinukleotide erforderlich (Vgl.: Herman et al.: Methylation-specific PCR: a novel PCR assay for methylation status of CpG islands. Proc Natl Acad Sci U S A. 1996 Sep 3;93(18):9821-6).

Eine andere ganz besonders bevorzugten Ausführungsform ist für Bisulfitvorbehandelte DNA unter dem Namen "Heavy-Methyl"-Methode bekannt. Dabei wird eine spezifische Amplifizierung nur der ursprünglich methylierten (bzw. unmethylierten) DNA durch Einsatz mindestens eines methylierungsspezifischen Blocker-Oligomers erreicht. Der Blocker bindet an ein 5'-CG-3' (bzw. 5'-TG-3'-Dinukleotid oder 5'-CA-3')-Dinukleotid und verhindert so die Amplifikation der Hintergrund-DNA. Die Ausführungsform kann über die Auswahl der Polymerase oder über die Modifikation der Blockeroligomere so ausgestaltet sein, daß ein Abbau oder eine Verlängerung der Blocker minimiert wird (zur Übersicht: WO 02/072880; Cottrell et al., A real-time PCR assay for DNA-methylation using methylation-specific blockers. Nucleic Acids Res. 2004 Jan 13;32(1):e10.).

Die Detektion der Amplifikate kann über herkömmliche Verfahren erfolgen, etwa über Methoden der Längenmessung wie Gelelektrophorese, Kapillargelelektrophorese und Chromatographie (z.B. HPLC). Auch Massenspektrometrie und Methoden zur Sequenzierung wie die Sanger-Methode, die Maxam-Gilbert-Methode und Sequencing by Hybridisation (SBH) können verwendet werden. In einer bevorzugten Ausführungsform werden die Amplifikate durch Primer-Extension-Verfahren nachgewiesen (siehe etwa: Gonzalgo & Jones: Rapid quantitation of methylation differences at specific sites using methylation-sensitive single nucleotide primer extension (Ms-SNuPE). Nucleic Acids Res. 1997 Jun 15;25(12):2529-31; DE 100 10 282; DE 100 10 280).

In einer anderen bevorzugten Ausführungsform werden die die Amplifikate mittels Hybridisierung an Oligomer-Arrays analysiert (ein Überblick über Array-Technologie befindet sich in der Extraausgabe von: Nature Genetics Supplement, Volume 21, January 1999). Auf einem solchen Array können die verschiedenen Oligomere auf einer Festphase in Form eines rechtwinkligen oder hexagonalen Gitters angeordnet sein. Die Festphasenoberfläche ist bevorzugt aus Silizium, Glas, Polystyrol, Aluminium, Stahl, Eisen, Kupfer, Nickel, Silber oder Gold zusammengesetzt. Jedoch sind auch Nitrocellulose und Kunststoffe wie Nylon, die in Form von Pellets oder auch als Harz-Matrizes existieren können, möglich. Die etwa fluoreszenzmarkierten Amplifikate werden an die gebundenen Oligomers hybridisiert und die nicht gebundenen Fragmente entfernt. Vorteilhaft ist es dabei, wenn die Oligomere über einen 12-22 Basen langen Abschnitt an die zu analysierende DNA hybridisieren und sie mindestens ein CG, TG oder CA Dinukleotid umfassen. Die Fluoreszenz-Signale können gescannt und mit Software-Programmen verarbeitet werden (Siehe etwa: Adorjan et al., Tumour class prediction and discovery by microarray-based DNA methylation analysis. Nucleic Acids Res. 2002 Mar 1;30(5):e21).

Ganz besonders bevorzugt werden die Amplifikate unter Verwendung von PCR-Real-Time-Varianten analysiert (vgl.: Heid et al.: Real time quantitative PCR. Genome Res. 1996 Oct;6(10):986-94, US Patent No. 6,331,393 "Methyl-Light"). Dabei wird die Amplifikation in Gegenwart eines fluoreszenzmarkierten Reporteroligonukleotid durchgeführt, das an ein 5'-CG-3'-Dinukleotid (bzw. 5'-TG-3'- oder 5'-CA-3'-Dinukleotid) hybridisiert. Das Reporteroligonukleotid bindet dabei bevorzugt an die zu untersuchende DNA und zeigt deren Amplifikation durch Zunahme oder Abnahme der Fluoreszenz an. Dabei ist es besonders vorteilhaft, wenn die Fluoreszenzveränderung direkt zur Analyse benutzt wird und aus dem Fluorenzenzsignal auf einen Methylierungszustand geschlossen wird. Eine besonders bevorzugte Variante ist dabei das "Taqman"-Verfahren. In einer anderen besonders bevorzugten Ausführungsform wird ein zusätzliches floureszenzmarkiertes Oligomer verwendet, das in unmittelbarer Nähe zu dem ersten Reporteroligonukleotid hybridisiert und sich diese Hybridisierung mittels Fluoreszenz-Resonanz-Energietransfer nachweisen läßt ("Lightcycler"-Verfahren).

Eine andere bevorzugte Ausführungsform der Erfindung ist es, mehrere Fragmente gleichzeitig mittels einer Multiplex-PCR zu amplifizieren. Bei deren Design muss darauf geachtet werden, daß nicht nur die Primer, sondern auch die weiteren eingesetzten Oligonukleotide nicht zueinander komplementär sein dürfen, so daß eine hochgradige Multiplexierung in diesem Fall schwieriger ist als in üblich. Jedoch hat man bei der enzymatisch vorbehandelten DNA den Vorteil, daß aufgrund des unterschiedlichen G und C-Gehaltes der beiden DNA-Stränge ein Forward-Primer niemals auch als Reverse-Primer fungieren kann, was die Multiplexierung wiederum erleichtert und den oben beschriebenen Nachteil im wesentlichen ausgleicht. Die Detektion der Amplifikate ist wiederum über unterschiedliche Verfahren möglich. Denkbar ist dabei etwa die Verwendung von Real-Time-Varianten. Für Amplifikationen von mehr als vier Genen empfiehlt es sich aber, die Amplifikate auf andere Weise zu detektieren. Bevorzugt ist dabei eine Analyse über Arrays (s.o.).

Im übrigen wird noch einmal betont, daß alle bekannten Verfahren zur Analyse bisulfit-umgewandelter DNA auch erfindungsgemäß eingesetzt werden können. Der Fachmann findet Angaben über die entsprechenden Verfahren in der wissenschaftlichen Veröffentlichungen und in der Patentliteratur. Eine aktuelle Übersicht über die mögliche Methoden findet sich in: Fraga and Esteller: DNA Methylation: A Profile of Methods and Applications. Biotechniques 33:632-649 (September 2002). Ganz besondere Vorteile des erfindungsgemäßen Verfahrens ergeben sich allerdings - wie oben beschrieben - in Kombination mit Verfahren zur sensitiven Detektion von Methylierungsmustern, also insbesondere bei Anwendung von methylierungsspezifischen PCR-Amplifikationen in Kombination Real Time Detektions Verfahren.

Eine besonders bevorzugte Verwendung des erfindungsgemäßen Verfahrens liegt in der Diagnose von Krebserkrankungen oder anderen mit einer Veränderung des Methylierungsstatus assoziierten Krankheiten. Hierzu gehören u.a. CNS-Fehlfunktionen, Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Das erfindungsgemäße Verfahren eignet sich außerdem zur Vorhersage von unerwünschten Arzneimittelwirkungen und zur Unterscheidung von Zelltypen oder Geweben oder zur Untersuchung der Zelldifferenzierung.

Erfindungsgemäß ist schließlich auch ein Kit, der aus mindestens einem methylierungssensitiven Restriktionsenzym und aus Reagenzien für eine Bisulfitumwandlung besteht, sowie optional auch eine Polymerase, Primer und Sonden für eine Amplifkation und Detektion enthält.

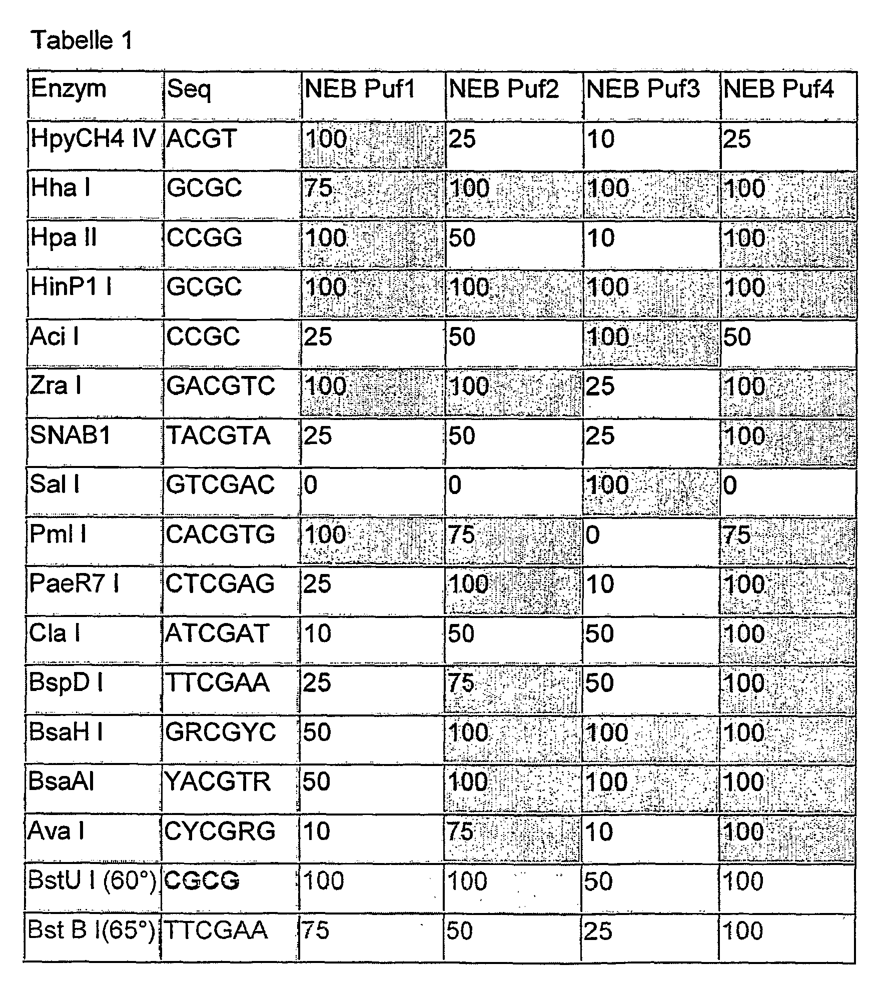

Tabelle 1 zeigt die Restriktionsstellen unterschiedlicher Enzyme, die für das erfindungsgemäße Verfahren einsetzbar sind. Die Tabelle ist dem Katalog von New England Biolabs entnommen (www.neb.com). Die Enzyme haben ihr Optimum in unterschiedlichen Puffern (NEB-Puffer 1-4). Angegeben ist die relative Aktivität der Enzyme in %. Die schraffierten Flächen zeigen die Puffer, bei denen die Enzyme am besten eingesetzt werden. In einer bevorzugten Ausführungsform der Erfindung werden mehrere Enzyme zusammen eingesetzt. Dabei werden bevorzugt solche Enzyme kombiniert, die ihr Optimum bei den gleichen Pufferkonzentrationen haben.

### Beispiel 1

Es soll ein diagnostischer Test entwickelt werden, mit dem aus einer Blutprobe sehr frühzeitig Lebererkrankungen, insbesondere Leberkrebs, nachgewiesen werden kann. Dazu wird eine DNA-Sequenz untersucht, die nur in Lebergewebe methyliert ist, in anderen Geweben (z.B. Muskel, Lunge, Haut, Brust) aber unmethyliert vorliegt (siehe unten, vgl.: DE 100 32 529). Kann man in Blut die spezifisch methylierte Sequenz nachweisen, so ist dies ein Hinweis darauf, dass das Lebergewebe geschädigt ist. Ein technisches Problem besteht jedoch darin, dass neben der spezifisch methylierten DNA eine hohe Menge an unmethylierter DNA derselben Sequenz vorliegt. Um einen spezifischen Test zu entwickeln, ist es daher sehr vorteilhaft, die Hintergrund DNA zunächst spezifisch abzubauen. Dazu wird - wie unten aufgeführt - eine Kombination aus drei Restriktionsenzymen verwendet. Anschließend wird die DNA einer Bisulfitumwandlung unterzogen, und die umgewandelte DNA wird methylierungsspezifisch amplifiziert. Anhand der Anwesenheit oder der Menge der Amplifikate kann dann auf das Vorliegen einer Krankheit geschlossen werden.

Hierzu wird zunächst die DNA aus Blut des Probanten isoliert. Hierzu stehen unterschiedliche Verfahren zur Verfügung, etwa unter Verwendung des Qiagen "UltraSens DNA Extraktions-Kits".
Anschließend werden die drei Restriktionsenzyme HpyCH4 IV (Schnittstelle:ACGT), Aci I (CGCC/GGCG) und Hpall(CCGG) nach Herstellerangaben eingesetzt. Schließlich wird die DNA in Gegenwart denaturierender Lösemittel unter Verwendung besondere Temperaturprogramme bisulfitiert und bevorzugt über eine Ultrafiltration aufgereinigt (siehe im einzelnen die deutschen Patentanmeldungen DE 103 47 396.3; DE 103 47 397.1; DE 103 47 400.5 und DE 103 47 399.8). Anschließend wir die umgewandelte DNA mittels zweier methylierungsspezifischer Primer amplifiziert (s.u.). Die Amplifikate werden bevorzugt mit Hilfe von Real-Time-Sonden detektiert (vgl.: "MethyLight" - WO00/70090; US 6,331,393).

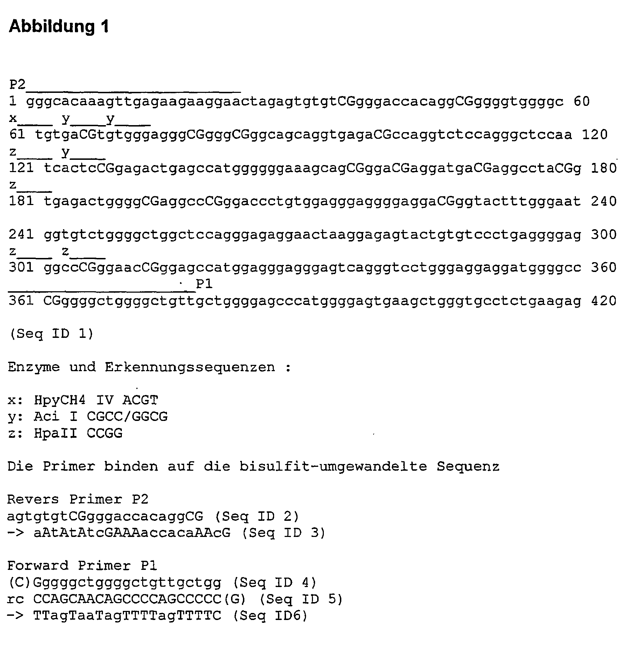

Die Sequenzen des Markers und die Schnittstellen der Enzyme sind in Abbildung 1 dargestellt.

### Beschreibung der Abbildungen

### Abbildung 1

Abbildung zeigt die Sequenz des Lebermarkers, die Schnittstellen der verwendeten Restriktionsenzyme und die Bindungsstellen der Primer (bindend auf der umgewandelten Sequenz). Es sind unterschiedliche Primer gezeigt, je nachdem welcher der umgewandelten (nicht mehr komplementären) DNA-Stränge amplifiziert werden soll. Untersucht wurde ein Lebermarker, der bereits in der deutschen Patentanmeldung DE 100 32 529 beschrieben ist.

## Patentansprüche

1. Verfahren zur Untersuchung von Cytosin-Methylierung zur Diagnose von Krebserkrankungen oder anderen mit einer Veränderung des Methylierungsstatus assoziierten Krankheiten, **dadurch gekennzeichnet, dass** folgende Schritte durchgeführt werden:
a) aus einer Gewebeprobe oder aus einer Körperflüssigkeit wird DNA isoliert,
b) die DNA wird mit Hilfe mindestens eines methylierungsspezifischen Restriktionsenzyms umgesetzt, wobei das methylierungsspezifische Restriktionsenzym die Hintergrund DNA abbaut,
c) die DNA wird chemisch oder enzymatisch umgewandelt, wobei unmethyliertes Cytosin in Uracil oder eine andere Base überführt wird, die sich im Basenpaarungsverhalten von Cytosin unterscheidet, während Methylcytosin unverändert bleibt,
d.) Analyse der umgewandelten DNA mittels real-time PCR

2. Verfahren nach Anspruch 1, **dadurch gekennzeichnet, dass** im zweiten Schritt eines der folgenden Enzyme verwendet wird: HpyCH4 IV, Hha I, Hpa II; HinP1I; Aci I, Zra I, SNAB1, Sal I; Pml1, PaeR7I, Cla I, BspDI, BsaAI, Ava I.

3. Verfahren nach einem der Ansprüche 1-2, **dadurch gekennzeichnet, dass** im zweiten Schritt eine Mischung unterschiedlicher Enzyme eingesetzt wird.

4. Verfahren nach Anspruch 3, **dadurch gekennzeichnet, dass** die unterschiedlichen eingesetzten Enzyme bei denselben Puffer- und Reaktionsbedingungen aktiv sind.

5. Verfahren nach einem der Ansprüche 1-4, **dadurch gekennzeichnet, dass** die Umwandlung im dritten Schritt mittels eines Bisulfits erfolgt.

6. Verfahren nach einem der Ansprüche 1-4, **dadurch gekennzeichnet, dass** die Umwandlung im dritten Schritt mittels einer methylierungsspezifischen Cytidin-Deaminase erfolgt.

7. Verfahren nach Anspruch 1, **dadurch gekennzeichnet, dass** die Polymerasekettenreaktion mittels methylierungspezifischer Primer erfolgt.

8. Verfahren nach dem Anspruch 7 **dadurch gekennzeichnet, dass** in der Polymerasekettenreaktion mindestens ein methylierungsspezifisches Blocke-Oligomer eingesetzt wird.

9. Verfahren nach den Ansprüchen 7 und 8 **dadurch gekennzeichnet, daß** die Amplifikate über Methoden der Längenmessung, der Massenspektrometrie oder der Sequenzierung analysiert werden.

10. Verfahren nach den Ansprüchen 7-9 **dadurch gekennzeichnet, daß** die Amplifikate durch Primer-Extension-Verfahren analysiert werden.

11. Verfahren nach den Ansprüchen 7-9 **dadurch gekennzeichnet, daß** die Amplifikate durch Hybridisierung an Oligomer-Arrays analysiert werden.

12. Verfahren nach Anspruch 1, **dadurch gekennzeichnet, daß** ein Taqman- oder ein Lightcycler-Verfahren durchgeführt wird.

13. Verfahren nach den Ansprüchen 7-12, **dadurch gekennzeichnet, daß** mehrere Fragmente gleichzeitig mittels einer Multiplex-Reaktion amplifiziert werden.

14. Verwendung der Verfahren nach Ansprüchen 1-13 zur Vorhersage von unerwünschten Arzneimittelwirkungen, zur Unterscheidung von Zelltypen und Geweben oder zur Untersuchung der Zelldifferenzierung.

## Claims

1. A method for the examination of cytosine methylation for the diagnosis of cancer or other diseases associated with a change of the methylation status, **characterized in that** the following steps are carried out:
a) DNA is isolated from a tissue sample or from a body fluid,
b) the DNA is reacted with the aid of at least one methylation-specific restriction enzyme, wherein the methylation-specific restriction enzyme degrades the background DNA,
c) the DNA is chemically or enzymatically converted, wherein non-methylated cytosine is converted into uracil or another base that differs in the base pairing behavior from cytosine whereas methylcytosine remains unchanged,
d) analysis of the transformed DNA by real-time PCR.

2. The method according to Claim 1, **characterized in that** one of the following enzymes is used in the second step: HpyCH4 IV, Hha I, Hpa II; HinP1I; Aci I, Zra I, SNAB1, Sal I; Pmi1, PaeR7I, Cla I, BspDI, BsaAI, Ava I.

3. The method according to any one of Claims 1-2, **characterized in that** a mixture of different enzymes is used in the second step.

4. The method according to Claim 3, **characterized in that** the different enzymes used are active under the same buffer and reaction conditions.

5. The method according to any one of Claims 1-4, **characterized in that** the conversion takes place in the third step by means of a bisulfite.

6. The method according to any one of Claims 1-4, **characterized in that** the conversion takes place in the third step by means of a methylation-specific cytidine deaminase.

7. The method according to Claim 1, **characterized in that** the polymerase chain reaction takes place by means of methylation-specific primers.

8. The method according to Claim 7, **characterized in that** at least one methylation-specific blocker oligomer is used in the polymerase chain reaction.

9. The method according to the Claims 7 and 8, **characterized in that** the amplificates are analyzed via methods of the longitudinal measuring, the mass spectrometry or the sequencing.

10. The method according to the Claims 7-9, **characterized in that** the amplificates are analyzed by primer-extension methods.

11. The method according to the Claims 7-9, **characterized in that** the amplificates are analyzed by hybridization on oligomer arrays.

12. The method according to Claim 1, **characterized in that** a TaqMan method or a lightcycler method is carried out.

13. The method according to the Claims 7-12, **characterized in that** several fragments are amplified simultaneously by means of a multiplex reaction.

14. The use of the methods according to Claims 1-13 for the prediction of undesired drug effects, for distinguishing cell types and tissues or for the examination of the cell differentiation.

## Revendications

1. Méthode pour l'analyse de la méthylation de cytosine pour le diagnostic de maladies cancéreuses ou autres maladies associées à une modification de l'état de méthylation, **caractérisé en ce que** les étapes suivantes sont réalisées :
a) l'ADN est isolée d'un échantillon de tissu ou d'un liquide organique,
b) l'ADN est converti à l'aide d'au moins un enzyme de restriction spécifique à la méthylation, l'enzyme de restriction spécifique à la méthylation décomposant l'ADN d'arrière-plan,
c) l'ADN est transformée chimiquement ou enzymatiquement, de la cytosine non méthylée est transformée en Uracil ou une autre base qui se distingue de la cytosine en ce qui concerne son comportement dans l'appariement de bases, tandis que la méthylcytosine reste inchangée,
d) analyse de l'ADN transformée au moyen de PCR en temps réel.

2. Méthode selon la revendication 1, **caractérisé en ce que** l'on utilise à la deuxième étape les enzymes suivants : HpyCH4 IV, Hha I, Hpa II ; HinP1I ; Aci I, Zra I, SNAB1, Sal I ; Pml1, PaeR7I, Cla I, BspDI, BsaAI, Ava I.

3. Méthode selon l'une quelconque des revendications 1-2, **caractérisé en ce que** l'on utilise dans la deuxième étape un mélange d'enzymes différents :

4. Méthode selon la revendication 3, **caractérisé en ce que** les différents enzymes utilisés sont actifs dans les mêmes conditions de tampon et de réaction.

5. Méthode selon l'une quelconque des revendications 1-4, **caractérisé en ce que** la transformation à la troisième étape se fait au moyen d'un bisulfite.

6. Méthode selon l'une quelconque des revendications 1-4, **caractérisé en ce que** la transformation à la troisième étape se fait au moyen d'une cytidine-déaminase spécifique à la méthylation.

7. Méthode selon la revendication 1, **caractérisé en ce que** la réaction en chaîne de polymérases se fait au moyen d'une amorce spécifique à la méthylation.

8. Méthode selon la revendication 7, **caractérisé en ce que** l'on utilise dans la réaction en chaîne de polymérases au moins un oligomère bloquant spécifique à la méthylation.

9. Méthode selon les revendications 7 et 8, **caractérisé en ce que** les amplificateurs sont analysés à l'aide de méthodes de mesure de longueur, de la spectrométrie de masse ou du séquençage.

10. Méthode selon les revendications 7-9, **caractérisé en ce que** les amplificateurs sont analysés par le méthode d'extension d'amorce.

11. Méthode selon les revendications 7-9, **caractérisé en ce que** les amplificateurs sont analysés par hybridation sur des séries d'oligomères.

12. Méthode selon la revendication 1, **caractérisé en ce que** l'on réalise un méthode Taqman ou un méthode Lightcycler.

13. Méthode selon les revendications 7-12, **caractérisé en ce que** plusieurs fragments sont amplifiés simultanément au moyen d'une réaction multiplex.

14. Utilisation des méthodes selon les revendications 1-13 pour la prédiction d'effets indésirables de médicaments, pour la distinction de types de cellules et de tissus ou pour l'analyse de la différentiation cellulaire.


### SEQUENZPROTOKOLL

<110> Epigenomics AG
<120> Verfahren zur Untersuchung von Cytosin-Methylierungen in DNA
<130> P329105PCT
<140>
   <141>
<150> 102005011398.2
   <151> 2005-03-03
<160> 6
<170> PatentIn Ver. 2.1
<210> 1
   <211> 420
   <212> DNA
   <213> Homo sapiens
<400> 1
   gggcacaaag ttgagaagaa ggaactagag tgtgtcgggg accacaggcg ggggtggggc 60
   tgtgacgtgt gggagggcgg ggcgggcagc aggtgagacg ccaggtctcc agggctccaa 120
   tcactccgga gactgagcca tggggggaaa gcagcgggac gaggatgacg aggcctacgg 180
   tgagactggg gcgaggcccg ggaccctgtg gagggagggg aggacgggta ctttgggaat 240
   ggtgtctggg gctggctcca gggagaggaa ctaaggagag tactgtgtcc ctgaggggag 300
   ggcccgggaa ccgggagcca tggagggagg gagtcagggt cctgggagga ggatggggcc 360
   cgggggctgg ggctgttgct ggggagccca tggggagtga agctgggtgc ctctgaagag 420
<210> 2
   <211> 22
   <212> DNA
   <213> Künstliche Sequenz
<220>
   <223> Beschreibung der künstlichen Sequenz: Primer
<400> 2
   agtgtgtcgg ggaccacagg cg 22
<210> 3
   <211> 22
   <212> DNA
   <213> Künstliche Sequenz
<220>
   <223> Beschreibung der künstlichen Sequenz: Primer
<400> 3
   aatatatcga aaaccacaaa cg 22
<210> 4
   <211> 21
   <212> DNA
   <213> Künstliche Sequenz
<220>
   <223> Beschreibung der künstlichen Sequenz: Primer
<400> 4
   gggggctggg gctgttgctg g 21
<210> 5
   <211> 21
   <212> DNA
   <213> Künstliche Sequenz
<220>
   <223> Beschreibung der künstlichen Sequenz: Primer
<400> 5
   ccagcaacag ccccagcccc c 21
<210> 6
   <211> 21
   <212> DNA
   <213> Künstliche Sequenz
<220>
   <223> Beschreibung der künstlichen Sequenz: Primer
<400> 6
   ttagtaatag ttttagtttt c 21